# EUROPEAN PATENT APPLICATION

(11) **EP 4 563 069 A1**
(43) Date of publication of application: **04.06.2025**
(21) Application number: 23846022.4
(22) Date of filing: 06.06.2023
(51) Int. Cl.: A61B 1/12, G02B 23/24

(54) **PROCESSOR FOR ELECTRONIC ENDOSCOPE**

(30) Priority: 28.07.2022 JP 2022120378
(71) Applicant: HOYA CORPORATION, Shinjuku-ku Tokyo 160-8347 (JP)
(72) Inventor: HIJIKATA, Takao, Tokyo 160-8347 (JP)
(74) Representative: Schaumburg und Partner Patentanwälte mbB
(86) International application number: PCT/JP2023/021013
(87) International publication number: WO 2024/024286

(57) **Abstract**

One aspect of the present invention is an electronic endoscope processor including: a housing; an exhaust fan that is attached to the housing and exhausts air in an internal space of the housing to the outside; a light source unit that is arranged in the housing and emits illumination light for illuminating a living tissue; and a blowing fan that is provided near the light source unit in the housing and sends air in the periphery of the light source unit to the exhaust fan, An intake hole through which outside air is introduced toward the periphery of the light source unit is formed in the housing.

## Description

### Technical Field

The present invention relates to an electronic endoscope processor configured to acquire and process a captured image of a living tissue.

### Background Art

An electronic endoscope system is used to observe or treat a living tissue inside a human body. The electronic endoscope system includes an electronic endoscope that captures an image of a living tissue with an image sensor and transmits the captured image to a processor, and the processor (electronic endoscope processor) that processes a signal of the captured image to create an image for display.

In the electronic endoscope processor, a light source device for observing a living tissue may be built in the processor. In this case, a measure for mitigating the influence on cooling performance of the light source device due to a temperature rise in a housing caused by a heat source inside the processor is required.

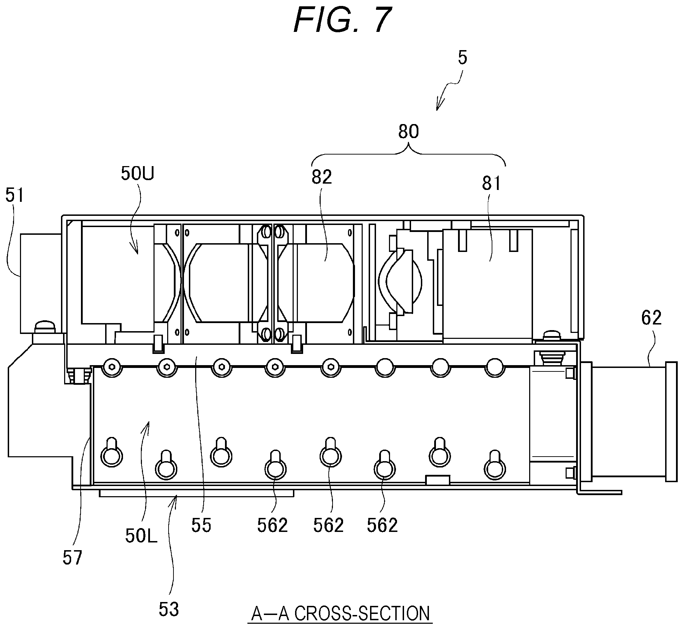

For example, US 2013/0188388 A describes a solid state illumination system in which a housing is divided into upper and lower spaces by a platform with fins extending downward, and a light source and an optical system are arranged in the upper space and a cooling system including a fan is arranged in the lower space in order to reduce a possibility that components of the optical system are contaminated by an air flow (see Figs. 7A to 7D). In the lower space, the fan is configured to draw an air flow from an aperture on an opposite side of the fan and exhaust air to release heat.

### Summary of Invention

### Technical Problem

In the light source device (the foregoing solid state illumination system) described in US 2013/0188388 A, the fan is configured to draw and exhaust the air flow through the aperture on the opposite side of the fan in the housing. Therefore, in a case where the light source device described in US 2013/0188388 A is mounted in a housing of an electronic endoscope processor, air in the housing is taken into the aperture, but since there is a heat source other than the light source device in the housing, the temperature is higher than room temperature, the light source device is cooled at a temperature higher than room temperature, and cooling efficiency is poor.

Therefore, an object of the present invention is to enable efficiently cooling of a light source device in a case where the light source device is built in an electronic endoscope processor.

### Solution to Problem

One aspect of the present invention is an electronic endoscope processor including:
a housing;
an exhaust fan that is attached to the housing and exhausts air in an internal space of the housing to the outside;
a light source unit that is arranged in the housing and emits illumination light for illuminating a living tissue; and
a blowing fan that is provided near the light source unit in the housing and sends air in the periphery of the light source unit to the exhaust fan.
An intake hole through which outside air is introduced toward the periphery of the light source unit is formed in the housing.

The electronic endoscope processor may include a heat transfer structure portion that is connected to the light source unit in the housing, has an internal space, and transfers heat generated by the light source unit. In this case, outside air is directly introduced into the internal space of the heat transfer structure portion from the intake hole.

The blowing fan may be attached to the heat transfer structure portion to send air in the internal space of the heat transfer structure portion to the exhaust fan.

It is preferable that the heat transfer structure portion is arranged at a bottom portion of the housing, and the intake hole is formed in the bottom portion of the housing.

It is preferable that the intake hole is hermetically sealed from an internal space other than the heat transfer structure portion in the housing.

The heat transfer structure portion may substantially include a plurality of fins extending along a direction from the intake hole toward the blowing fan.

The electronic endoscope processor may further include:
a temperature sensor attached to the light source unit or the heat transfer structure portion; and
a control unit that controls a rotational speed of the exhaust fan and/or the blowing fan based on a detection value of the temperature sensor.

The electronic endoscope processor may include a housing portion that hermetically houses the light source unit. In this case, it is preferable that the heat dissipation structure portion and the housing portion are integrated.

### Advantageous Effects of Invention

According to the above-described electronic endoscope processor, the light source device can be efficiently cooled in the case where the light source device is built in the electronic endoscope processor.

### Brief Description of Drawings

Fig. 1 is a block diagram illustrating an overall configuration of an electronic endoscope system of an embodiment.
Fig. 2 is a top view and a side view of the electronic endoscope processor according to the embodiment.
Fig. 3 is a bottom view of the electronic endoscope processor according to the embodiment.
Fig. 4 is a view of the inside of the electronic endoscope processor according to the embodiment as viewed from above.
Fig. 5 is a perspective view of a light source device mounted on the electronic endoscope processor according to the embodiment.
Fig. 6 is a perspective view of the light source device mounted on the electronic endoscope processor according to the embodiment as viewed from a different viewpoint from Fig. 5.
Fig. 7 is a view illustrating a cross-section taken along line A-A of Fig. 4 of the light source device mounted on the electronic endoscope processor according to the embodiment.
Fig. 8 is a perspective view of a heat discharge unit included in the light source device of Fig. 5.
Fig. 9 is a view for describing a cooling operation inside the electronic endoscope processor according to the embodiment.
Fig. 10 is a view illustrating the electronic endoscope processor including a temperature sensor.

### Description of Embodiments

### (System Configuration)

Hereinafter, an electronic endoscope system according to an embodiment will be described.

An electronic endoscope system according to the embodiment includes: an electronic endoscope (electronic scope) including an image sensor that acquires a captured image of a living tissue; and an electronic endoscope processor detachably connected to the electronic endoscope. The electronic endoscope processor is an electronic device that performs signal processing on the captured image of the living tissue to create an image for display.

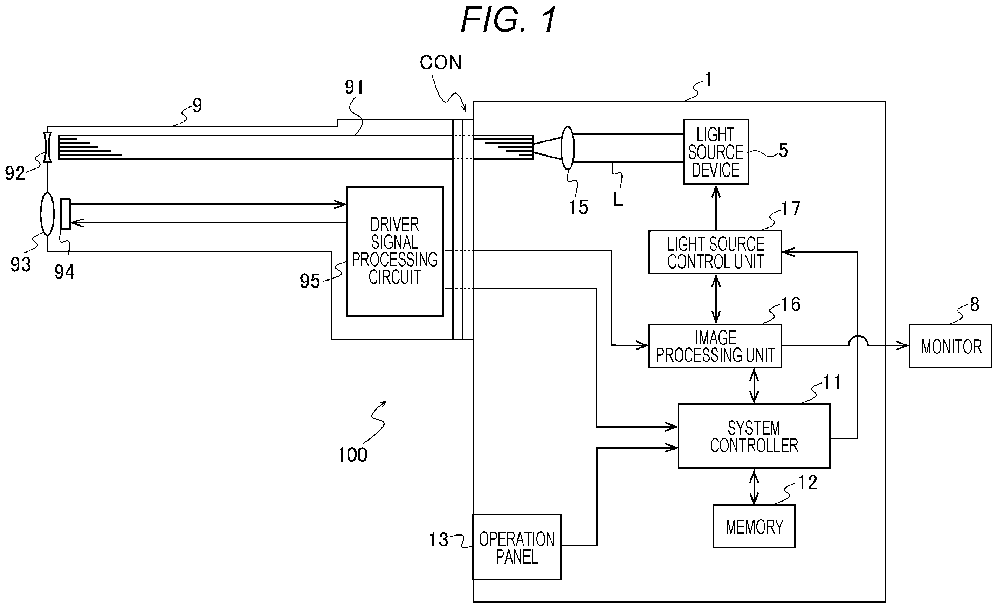

Fig. 1 illustrates a schematic configuration of an electronic endoscope system 100 according to the embodiment. As illustrated in Fig. 1, the electronic endoscope system 100 includes an electronic endoscope processor 1, an electronic scope 9, and a monitor 8. The electronic endoscope processor 1 and the electronic scope 9 are connected by a connector CON.

The electronic endoscope processor 1 includes a system controller 11. The system controller 11 executes various programs stored in a memory 12 and integrally controls the entire electronic endoscope system 100. Further, the system controller 11 is connected to an operation panel 13. The system controller 11 changes operations of the electronic endoscope system 100 and a parameter for each of the operations in accordance with an operator's instruction input to the operation panel 13.

The electronic endoscope processor 1 includes a light source device 5. The light source device 5 emits illumination light L for illuminating an object such as a living tissue in a body cavity. A light source of the light source device 5 is, for example, a high luminance lamp (for example, a xenon lamp, a metal halide lamp, a mercury lamp, a halogen lamp, or the like) that emits white illumination light, a plurality of light emitting diodes emitting light in a wavelength band of a predetermined color, or a laser light source. The illumination light L emitted from the light source device 5 is condensed onto an incident end face of a light carrying bundle (LCB) 91 by a condenser lens 15, and is incident into the LCB 91.

The illumination light L incident into the LCB 91 propagates through the LCB 91**.** The illumination light L propagating through the LCB 91 is emitted from an exit end face of the LCB 91 arranged at a distal tip of the electronic scope 9, and is applied to the object via a light distribution lens 92**.** Return light from the object illuminated with the illumination light L from the light distribution lens 92 forms an optical image on a light receiving surface of a solid-state image sensor 94 via an objective lens 93**.**

The solid-state image sensor 94 is a single-plate color charge coupled device (CCD) image sensor having a Bayer pixel arrangement. The solid-state image sensor 94 accumulates an optical image formed by each of pixels on the light receiving surface, as charge corresponding to the amount of light, and generates and outputs image signals of Red (R), Green (G), and Blue (B). Note that the solid-state image sensor 94 is not limited to a CCD image sensor, and may be a complementary metal oxide semiconductor (CMOS) image sensor.

A driver signal processing circuit 95 is provided in a connection portion of the electronic scope 9. An image signal of the object is input to the driver signal processing circuit 95 from the solid-state image sensor 94 in a predetermined frame cycle. For example, the frame cycle is 1/30 seconds. The driver signal processing circuit 95 performs predetermined processing including A/D conversion on the image signal input from the solid-state image sensor 94 and outputs the processed image signal to an image processing unit 16 of the electronic endoscope processor 1**.**

The image processing unit 16 performs predetermined image processing to generate a video format signal, and outputs the video format signal to the monitor 8**.**

The electronic endoscope processor 1 includes a light source control unit 17 that acquires luminance information of the image signal from the image processing unit 16 and controls the intensity of the illumination light of the light source device 5 based on the luminance information.

The light source control unit 17 controls the light source device 5 such that the intensity of the illumination light emitted from the light source device 5 is high when brightness is low, and the intensity of the illumination light emitted from the light source device 5 is low when brightness is high. Accordingly, the brightness of the image signal received from the driver signal processing circuit 95 is controlled to be maintained constant.

The system controller 11 performs various calculations based on unique information of the electronic scope 9, and generates a control signal. The system controller 11 controls operations and timings of various circuits in the electronic endoscope processor 1 using the generated control signal such that processing suitable for the electronic scope 9 connected to the electronic endoscope processor 1 is performed.

### (Structure of Electronic Endoscope Processor 1)

Next, a structure of the electronic endoscope processor 1 will be described.

In the electronic endoscope processor 1, each of the system controller 11, the image processing unit 16, and the light source control unit 17 is a heat generating component configured by a circuit board including a central processing unit (CPU), an application specific integrated circuit (ASIC), a field-programmable gate array (FPGA), or the like. Therefore, the atmosphere around the light source device 5 inside a housing of the electronic endoscope processor 1 is higher than the room temperature, so that the light source device 5 cannot be efficiently cooled by air in the periphery of the light source device 5. Therefore, the electronic endoscope processor 1 according to the embodiment is configured to directly introduce outside air at room temperature into the light source device 5 in the housing as described later.

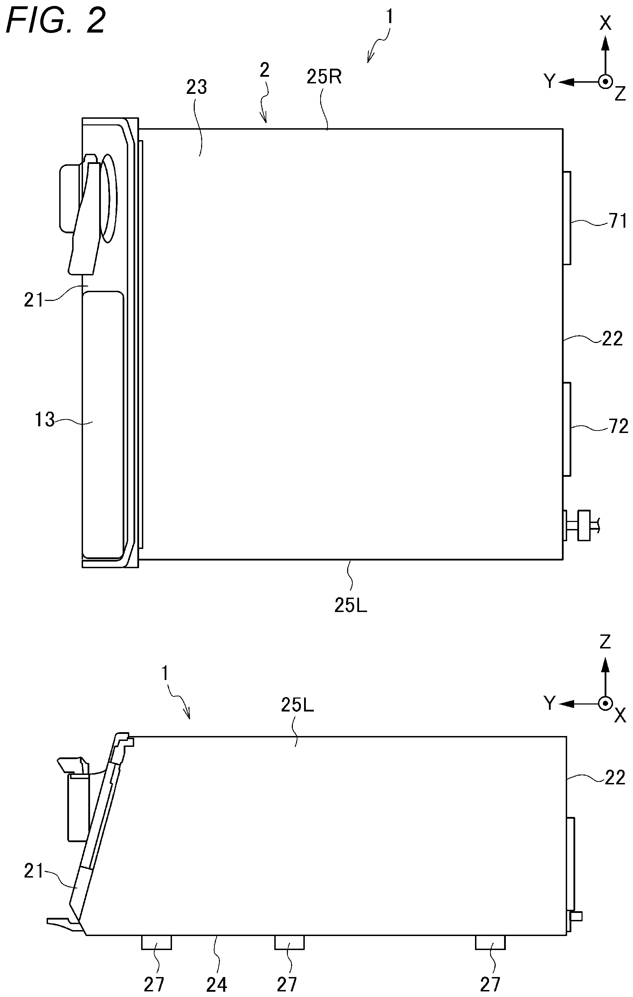

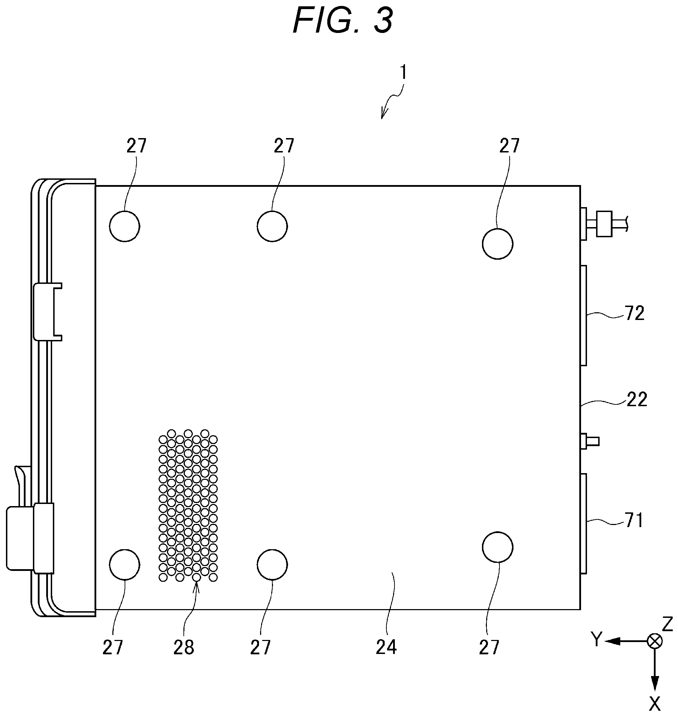

Fig. 2 illustrates a top view and a side view of the electronic endoscope processor 1 according to the embodiment. Fig. 3 illustrates a bottom view of the electronic endoscope processor 1 according to the embodiment.

As illustrated in Fig. 2, the electronic endoscope processor 1 includes a front surface portion 21, a rear surface portion 22, a top plate 23, a bottom plate 24 (an example of a bottom portion), a left side plate 25L, and a right side plate 25R, and has an appearance of a rectangular parallelepiped shape. A plurality of legs 27 are attached to the bottom plate 24. The operation panel 13 is arranged on the front surface portion 21. In the following description, the left side plate 25L and the right side plate 25R are described as a "side plate 25" when matters common to the left side plate 25L and the right side plate 25R are referred to.

Two exhaust fans 71 and 72 are installed on the rear surface portion 22 of the housing **2.** The exhaust fans 71 and 72 are provided to suck and discharge the air introduced into the housing 2.

Note that, in each drawing referred to in the following description, XYZ coordinate axes are described such that a direction from the left side plate 25L toward the right side plate 25R is a +X direction, a direction from the rear surface portion 22 toward the front surface portion 21 is a +Y direction, and a direction from the bottom plate 24 toward the top plate 23 is a +Z direction.

As illustrated in Fig. 3, an intake hole 28 is formed in the bottom plate 24 of the electronic endoscope processor 1. As described later, the intake hole 28 is provided to directly introduce the outside air at room temperature into the light source device 5. As illustrated in Fig. 2, the leg 27 is attached to the bottom plate 24, and a gap is formed between the bottom plate 24 and a placement surface of the electronic endoscope processor 1, so that the outside air can be introduced from the intake hole 28.

In the embodiment, the intake hole 28 is constituted by an assembly of a plurality of small holes. In the example illustrated in Fig. 3, a shape of each hole is circular, but is not limited thereto, and can be set to a shape close to a flat ellipse, a circle, a polygon, or the like.

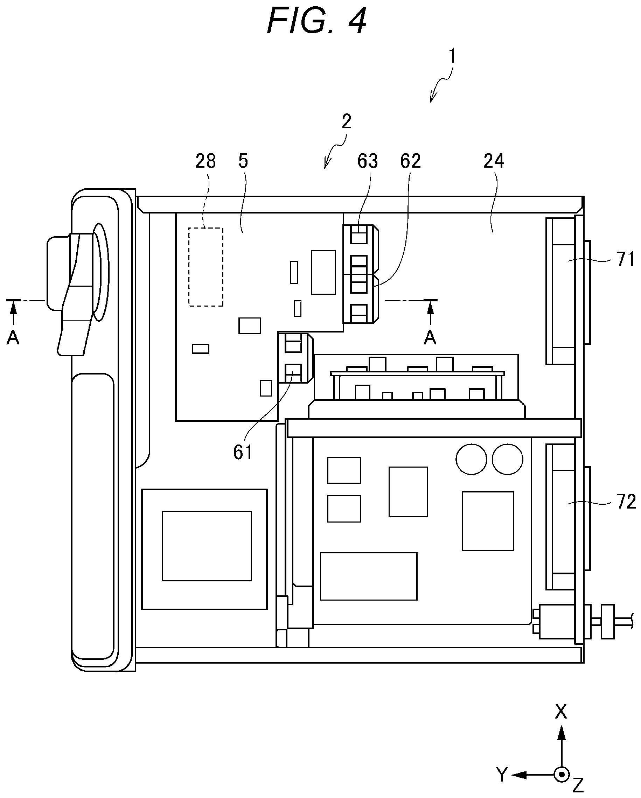

Fig. 4 is a view of the inside of the housing 2 of the electronic endoscope processor 1 according to the embodiment as viewed from above.

As illustrated in Fig. 4, the light source device 5 and a plurality of heat generating components are arranged on the bottom plate 24. The light source device 5 includes the light source built in an independent case, and includes three blowing fans 61 to 63 that send a high-temperature air flow in the case toward the exhaust fan 71. In order to efficiently direct the air flow blown out from the three blowing fans 61 to 63 to the exhaust fan 71, it is preferable that no component is arranged between each blowing fan and the exhaust fan 71.

As illustrated in Fig. 4, the intake hole 28 (see also Fig. 3) for introducing the outside air into the light source device 5 is formed in the bottom plate 24 of the housing 2 in which the light source device 5 is arranged.

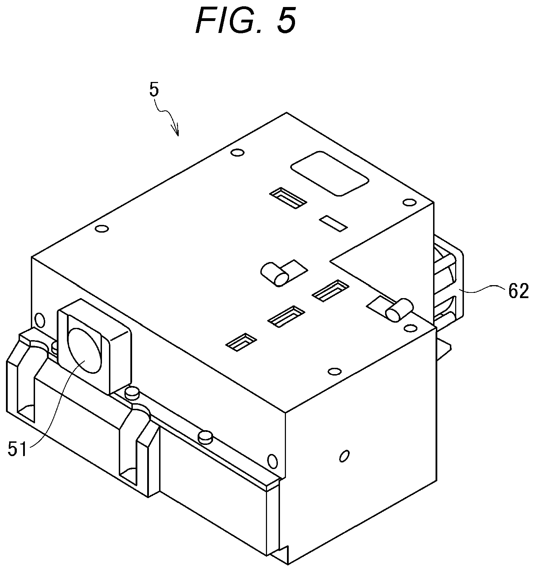

Next, the light source device 5 will be described with reference to Figs. 5 to 8.

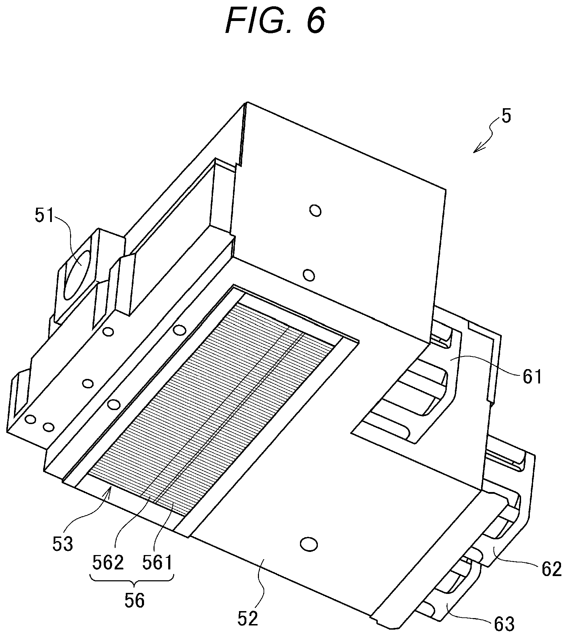

Fig. 5 is a perspective view of the light source device 5 mounted on the electronic endoscope processor 1 according to the embodiment as viewed from a viewpoint from which an upper surface is visible. Fig. 6 is a perspective view of the light source device 5 as viewed from a viewpoint from which the bottom surface is visible.

In the light source device 5, a light source group including a plurality of light sources and an optical system including a plurality of lenses are built in the case (housing). The light source device 5 is installed in the housing 2 such that a light emitting unit 51 faces forward (+Y direction).

The blowing fans 61 to 63 are provided at the rear of the light source device 5**,** that is**,** on the side opposite to the light emitting unit 51.

As illustrated in Fig. 6, an aperture 53 is formed in a bottom portion 52 of the light source device 5**.** The aperture 53 faces the intake hole 28 when the light source device 5 is arranged on the bottom plate 24 of the housing 2**,** and guides the outside air introduced from the intake hole 28 to the inside of the light source device 5.

Fig**.** 7 illustrates a cross-section taken along line A-A in Fig. 4 of the light source device 5 mounted on the electronic endoscope processor 1 according to the embodiment.

In the embodiment, an internal space of the light source device 5 is partitioned into a lower space 50L and an upper space 50U by a partition wall 55. In the upper space 50U, a light source unit 80 including a light source group 81 and an optical system 82 is arranged. A portion forming the upper space 50U of the light source device 5 is a housing portion that hermetically houses the light source unit 80. Therefore, contamination of the optical system 82 arranged in the upper space 50U is prevented. Light generated by the light source unit 80 is emitted from the light emitting unit 51.

In the embodiment, the light source device 5 includes a heat transfer structure portion 57. The heat transfer structure portion 57 includes the partition wall 55 and is connected to the light source unit 80. That is, the housing portion in which the light source unit 80 is housed and the heat transfer structure portion 57 are integrated.

The lower space 50L is formed in the heat transfer structure portion 57, and has a function of transferring heat generated by the light source unit 80. The heat transfer structure portion 57 is preferably made of, for example, metal (for example, aluminum, iron, or copper) having high thermal conductivity.

The outside air at room temperature is introduced into the lower space 50L through the intake hole 28 (see Fig. 2) and the aperture 53 of the housing 2. Therefore, the heat from the light source unit 80 transferred through the heat transfer structure portion 57 can be efficiently cooled by the outside air at room temperature.

The heat transfer structure portion 57 is provided with the blowing fans 61 to 63 that are provided near the light source unit 80 to send the air in the lower space 50L (air in the periphery of the light source unit 80) to the exhaust fan 71. The air warmed by the heat from the light source unit 80 is discharged from the lower space 50L of the light source device 5 and sent to the exhaust fan 71 by these blowing fans. Therefore, the heat generated by the light source unit 80 can be efficiently discharged from the light source device 5, and can be discharged from the housing 2 by the exhaust fan 71.

In the embodiment, a heat discharge unit 56 is provided in the lower space 50L formed by the heat transfer structure portion 57 in order to further enhance heat transfer performance of the heat transfer structure portion 57.

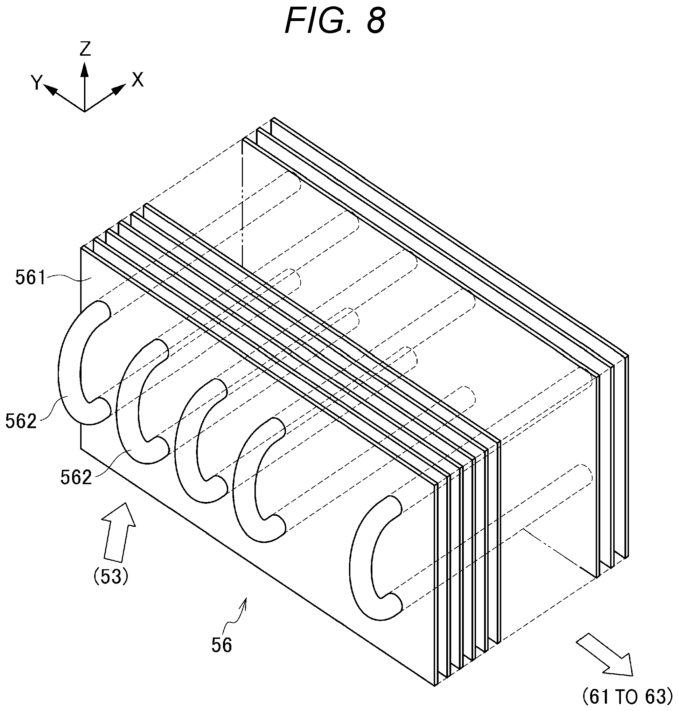

A configuration example of the heat discharge unit 56 is illustrated in Fig. 8. Fig. 8 is a perspective view of the exemplary heat discharge unit 56 included in the light source device 5. The heat discharge unit 56 substantially includes a heat sink 561 including a plurality of fins extending along a direction from the intake hole 28 and the aperture 53 toward the blowing fans 61 to 63. In the embodiment, the heat discharge unit 56 may have a heat pipe 562 as illustrated in Fig. 8. The heat sink 561 is connected to the partition wall 55 in order to enhance heat discharge performance from the light source unit 80.

In Fig. 8, a flow of air introduced from the aperture 53 toward the blowing fans 61 to 63 is indicated by an arrow. Each fin of the heat sink 561 is attached in a direction not to hinder the flow of air. That is, it is preferable that the flow of air toward the blowing fans 61 to 63 and a surface of each plate-shaped fin are substantially parallel.

Since the heat discharge unit 56 is provided, the efficiency of discharging the heat generated by the light source unit 80 can be further enhanced.

In the embodiment illustrated in Figs. 3 and 7, the heat transfer structure portion 57 of the light source device 5 is arranged on the bottom plate 24 of the housing 2, and the intake hole 28 is formed in the bottom plate 24 of the housing 2, whereby the outside air is introduced into the heat transfer structure portion 57 from the bottom portion of the housing **2.** This configuration has an advantage that it is convenient to introduce the outside air into the heat transfer structure portion 57 in a lower portion of the light source device 5 and mounting is easy. However, the present invention is not limited thereto, and the outside air may be introduced into the heat transfer structure portion 57 from a side portion of the housing 2 by providing an intake hole of the housing 2 in the side plate 25 and providing an aperture of the light source device 5 at a position corresponding to the intake hole.

In the embodiment, the intake hole 28 and the aperture 53 are hermetically sealed from an internal space other than the heat transfer structure portion 57 in the housing **2.** Since the intake hole 28 and the aperture 53 are hermetically sealed with packing or the like, air in the internal space other than the heat transfer structure portion 57 in the housing 2 is prevented from being introduced into the heat transfer structure portion 57. Since there are heat generating components other than the light source device 5 in the internal space other than the heat transfer structure portion 57 in the housing 2, the air in the internal space is higher than room temperature. Since the intake hole 28 and the aperture 53 are hermetically sealed, air higher than room temperature is prevented from entering the heat transfer structure portion 57, and a decrease in cooling efficiency is avoided. Further, unless the intake hole 28 and the aperture 53 are hermetically sealed, a wind speed of the outside air introduced from the intake hole 28 decreases, and there is a possibility that the cooling efficiency decreases.

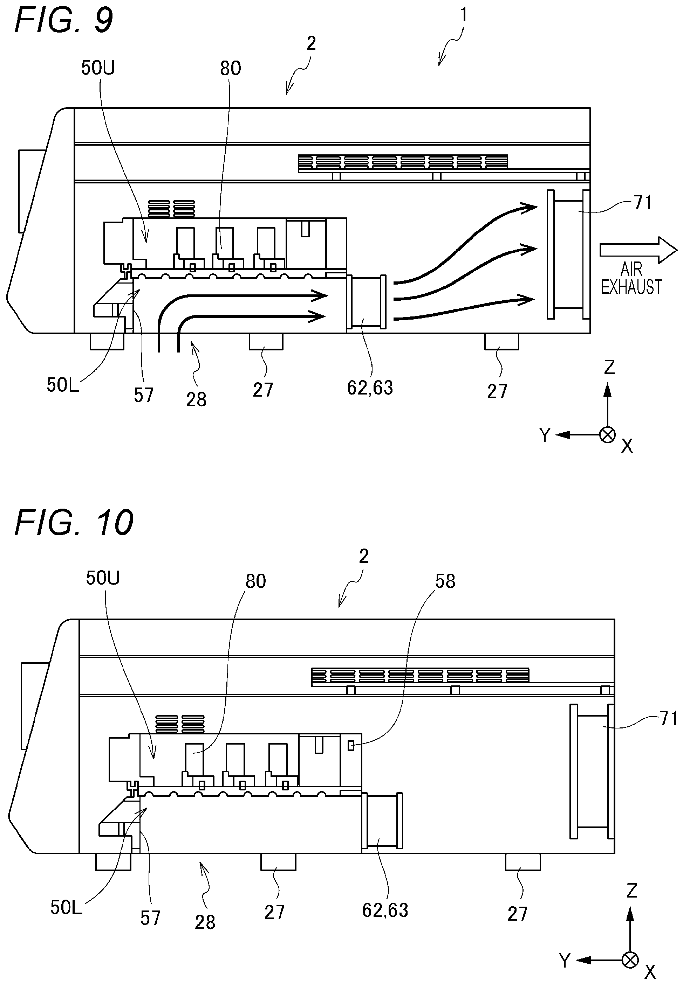

Next, a cooling operation of the electronic endoscope processor 1 will be described with reference to Fig. 9.

Fig. 9 is a view for describing the cooling operation inside the electronic endoscope processor 1 according to the embodiment. In Fig. 9, a flow of air introduced into and discharged from the housing 2 of the electronic endoscope processor 1 is indicated by arrows.

The light source unit 80 is arranged in the upper space 50U of the light source device 5. The light source device 5 includes the heat transfer structure portion 57 in which the lower space 50L is formed. Therefore, the heat generated by the operation of the light source unit 80 is transferred to the heat transfer structure portion 57 and discharged to the lower space 50L. Preferably, the heat discharge unit 56 (see Fig. 8) is arranged in the lower space 50L, and the heat is more efficiently discharged.

The outside air at room temperature is introduced into the lower space 50L through the intake hole 28 of the housing 2 and the aperture 53 of the light source device 5, and is sent to the exhaust fan 71 by the blowing fans 61 to 63. Therefore, the outside air warmed by the heat discharged by the heat transfer structure portion 57 is sent from the blowing fans 61 to 63 to the exhaust fan 71 and discharged to the outside of the housing 2. Since the blowing fans 61 to 63 and the exhaust fan 71 are arranged in series along the flow of air to increase a suction force, so that a large amount of outside air is acquired from the intake hole 28 of the housing 2. Therefore, a wind amount and a wind speed of an air flow from the intake hole 28 to the exhaust fan 71 increase, and the cooling performance can be improved. Since the wind amount and the wind speed of the air flow increase, it is also possible to suppress accumulation and adhesion of dust in the light source device 5 and in the housing 2 outside the light source device 5.

Note that the blowing fan and the exhaust fan may be of any type, and may be an axial fan or a blower fan.

In the embodiment, the electronic endoscope processor 1 may be provided with a temperature sensor 58 attached to the light source unit 80 or the heat transfer structure portion 57. Fig. 10 illustrates an arrangement example of the temperature sensor 58. In this example, the temperature sensor 58 is attached to the light source unit 80.

The system controller 11 (see Fig. 1; an example of a control unit) of the electronic endoscope processor 1 controls a rotational speed of the exhaust fan 71 and/or the blowing fans 61 to 63 based on a detection value of the temperature sensor 58. When the detection value of the temperature sensor 58 is higher than a predetermined value, it is considered that the cooling efficiency by the air flow passing through the heat transfer structure portion 57 is low. In this case, the system controller 11 may improve the cooling efficiency by increasing the rotational speed of the exhaust fan 71 and/or the blowing fans 61 to 63.

The electronic endoscope processor of the present invention has been described above in detail, but the electronic endoscope processor of the present invention is not limited to the foregoing embodiment, and may of course be modified or altered in various ways in a range not deviating from the scope and spirit of the present invention.

The present invention relates to a patent application of Japanese Patent Application No. 2022-120378 filed with the Japan Patent Office on July 28, 2022, the entire contents of which are incorporated herein by reference.

## Claims

1. An electronic endoscope processor comprising:
a housing;
an exhaust fan that is attached to the housing and exhausts air in an internal space of the housing to an outside;
a light source unit that is arranged in the housing and emits illumination light for illuminating a living tissue; and
a blowing fan that is provided near the light source unit in the housing and sends air in a periphery of the light source unit to the exhaust fan,
wherein an intake hole through which outside air is introduced toward the periphery of the light source unit is formed in the housing.

2. The electronic endoscope processor according to claim 1**,** further comprising
a heat transfer structure portion that is connected to the light source unit in the housing, has an internal space, and transfers heat generated by the light source unit,
wherein outside air is directly introduced into the internal space of the heat transfer structure portion from the intake hole.

3. The electronic endoscope processor according to claim **2,** wherein
the blowing fan is attached to the heat transfer structure portion to send air in the internal space of the heat transfer structure portion to the exhaust fan.

4. The electronic endoscope processor according to claim 2 or 3**,** wherein
the heat transfer structure portion is arranged at a bottom portion of the housing, and the intake hole is formed in the bottom portion of the housing.

5. The electronic endoscope processor according to claim 4**,** wherein
the intake hole is hermetically sealed from an internal space other than the heat transfer structure portion in the housing.

6. The electronic endoscope processor according to claim 2 or 3, wherein
the heat transfer structure portion substantially includes a plurality of fins extending in a direction from the intake hole toward the blowing fan.

7. The electronic endoscope processor according to claim 2 or 3, further comprising:
a temperature sensor attached to the light source unit or the heat transfer structure portion; and
a control unit that controls a rotational speed of the exhaust fan and/or the blowing fan based on a detection value of the temperature sensor.

8. The electronic endoscope processor according to claim 2 or 3, further comprising
a housing portion that hermetically houses the light source unit,
wherein the heat dissipation structure portion and the housing portion are integrated.
